Europäisches Patentamt

(19) European Patent Office

Office européen des brevets

(11) Publication number: **0 305 380 B1**

## (12) EUROPEAN PATENT SPECIFICATION

(45) Date of publication of patent specification :
25.09.91 Bulletin 91/39

(51) Int. Cl.⁵ : **A61K 31/78**

(21) Application number : **87902908.0**

(22) Date of filing : **20.03.87**

(86) International application number :
**PCT/US87/00584**

(87) International publication number :
**WO 88/06888 22.09.88 Gazette 88/21**

(54) NOVEL TOPICAL METRONIDAZOLE FORMULATIONS.

(43) Date of publication of application :
08.03.89 Bulletin 89/10

(45) Publication of the grant of the patent :
25.09.91 Bulletin 91/39

(84) Designated Contracting States :
AT BE CH DE FR GB IT LI LU NL SE

(56) References cited :
J.E.F. REYNOLDS et al.: "Martindale, the extra pharmacopoeia", 28th edition, 1982, pages 1452,1453, The Pharmaceutical Press, London, GB

(73) Proprietor : CURATEK PHARMACEUTICALS, INC.
1965 Pratt Boulevard Suite 101
Elk Grove Village, IL 60007 (US)

(72) Inventor : BORGMAN, Robert, John
1735 Victoria Road
Mundelein, IL 60060 (US)

(74) Representative : Heath, Derek James et al
BROMHEAD & CO. 19 Buckingham Street
London WC2N 6EF (GB)

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel topical formulations containing the drug metronidazole.

Metronidazole, 1-(2-hydroxyethyl)-2-methyl-5-nitroimidazole, is a drug known to be effective in treating a variety of disorders. For example, the drug has direct trichomonacidal and amebacidal activity against Trichomonas vaginalis and Entamoeba histolytica, and is useful in combatting infections caused by those microbial parasites. Metronidazole has also been reported to be effective (via both oral and topical application) in treating skin disorders such as rosacea, ulcers infected with anaerobic bacteria, including decubitus ulcers (bed or pressure sores), venous ulcers, and diabetic foot ulcers, and other anaerobic infections such as post operative sepsis. There have also been reports that metronidazole is effective against perioral dermatitis.

Although oral administration of the drug has been employed for the treatment of certain disorders, long-term oral administration of the drug in cases of chronic disorders such as rosacea may be associated with certain unwanted side effects, and subjects all organ systems needlessly to high drug concentrations. Well-known problems associated with systemic antibiotic therapy include gastro-intestinal intolerance and vaginitis. Thus, topical formulations are generally preferred for dermatological applications. (See "Practical Advice offered on Rosacea", Dermatology News, April, 1985).

It is known that the effectiveness of a drug against a particular illness may be profoundly affected by the vehicle into which the drug is incorporated. One difficulty in formulating topical compositions of metronidazole stems from the drug's low solubility in water and several other solvents. Accordingly, the topical preparations described to date for treatmnent of rosacea generally have been creams (oil in water emulsions) or ointments (petroleum jelly pased compositions). In these formulations, the drug is dissolved in the oil phase.

Rosacea, formerly called Acne rosacea ; is a chronic skin disease primarily affecting adults ; with recurring symptoms that include erythema, papules, pustules, rhinophyma, and telangiectses, primarily in the region of the nose, cheeks, and forehead. The metronidazole-containing topical formulations used to treat rosacea generally contain oils, certain surfactants and emulsifiers, and/or other ingredients which nave been found to be comedogenic, acnegenic, and/or irritating to the skin. (see Fulron et al., Amer. Acad. of Dermatology, Vol. 10, no. 1, pp. 96-105, (Jan. 1984)), Patients treated with such formulations therefore often experience skin problems which include irritation, uncomfortable drying of the skin, and "stinging" or "burning" sensations. In addition, the drug is generally dissolved or dispersed in the oil phase of such preparations, which reduces the specific activity of the drug due to inhibition of drug transfer across the cell membrane. Such effects are described in Nielsen, P., British J. of Dermatology, Vol. 108, pp. 327-332 (1983), for example.

Thus, a need remains for metronidazole-containing dermatological preparations suitable for topical use which avoid the problems of prior art formulations. Such dermatological preparations would be useful for treating skin disorders such as rosacea and certain types of dermatitis, including perioral dermatitis.

The aim of the present invention is to meet that need. In accordance with the present invention, therefore, a dermatological preparation in the form of an aqueous gel composition for topical application consists essentially of a) a therapeutically effective amount of metronidazole, b) a polycarboxylated vinyl polymer in an amount effective to promote solubility of the metronidazole and to cause gelling of the composition, and c) an aqueous solvent, and d) from 2% to 5% by weight of a penetration enhancer, wherein the preparation is substantially free of comodogenic, acneogenic, irritating and skin-drying ingredients.

The single-phase aqueous gels have higher specific activity of metronidazole compared to prior art oil-based formulations, due to increased bioavailability of the drug when solubilized in an aqueous composition. The compositions comprise non-comedogenic, non-irritating ingredients and thus avoid problems associated with the use of prior art formulations in the treatment of skin diseases. The aqueous gels of the present invention have properties which make them particularly suitable for the treatment of skin disorders such as rosacea and other acneform conditions and certain types of dermatitis.

Substantially oil-free, aqueous formulations of metronidazole, in which the drug is solubilized in a single-phase aqueous gel, are disclosed. The advantages of such aqueous formulations in treating skin disorders have been discussed above, and are presented in greater detail below.

When developing topical formulations for application to diseased skin, different aspects, such as thermodynamic activity of the drug in the vehicle (base), the release rate of the drug from the vehicle, the type and the status of the skin, and the sensitization and irritation potential of components are factors that can affect the therapeutic effectiveness of topical dermatological preparations. In the case of non-diseased skin with its intact stratum corneum, cell membrane-controlled penetration of the drug occurs, Therefore, a high thermodynamic activity of the drug in the vehicle is desirable (low affinity to the vehicle) to promote transfer of the drug across the epidermal cell membranes. With diseased skin, the release rate of the drug from the vehicle generally is rate-determining for penetration into a patient's cells. Therefore, vehicles which dissolve the drug and have a low diffusional resistance are preferred. In general, drug concentration in the vehicle, and thus the degree of saturation, is considered to be a key formulation factor when optimizing topical delivery for maximum bioavailability.

In rosacea (and other acneform conditions) and in certain types of dermatitis, topical formulations are usually applied to both normal and diseased areas. It is therefore desirable that a treatment have a mitigating effect on the diseased tissue and a prophylactic effect to present extension of involvement to the normal areas. Therefore, the most desired vehicle and hence formulation in these conditions should contain the drug in high thermodynamic activity (a high rate of cell membrane penetration) and with a fast rate of release from the vehicle. Aqueous formulations of metronidazole would appear to meet the above criteria. The low solubility of metronidazole in water (and in several other solvents), however, has resulted in the development of oil-based, rather than aqueous, netronidazole formulations for treatment of rosacea.

In the formulations of the present invention, metronidazole is dissolved in an aqueous solution of a high molecular weight polycarboxylated vinyl polymer. The polymer serves not only to promote solubility of the drug in water, but also imparts a desirable viscous, gelled consistency to the composition when mixed with the drug and water. The gels (> 95% water formulations) of the present invention have the requisite degree of metronidazole concentration and hence thermodynamic activity for effective topical delivery and bioavailability of the drug. The gels of the present invention also have the requisite therapeutic activities as described below. The present invention therefore provides compositions for the prophylactic or therapeutic treatment of humans afflicted with such skin disorders as rosacea, other acneform conditions (e.g., acne vulgaris, steriod acne, acne conglobata, or nodulocystic acne) or certain types of dermatitis (e.g., perioral dermatitis or seborrheic dermatitis).

The viscous gelled vehicle also minimizes "pooling" and "running" of the medication, e.g. pooling into facial creases, which sometimes occurs with dermatological cream preparations. The resulting local excesses of the creams may contribute to problematic erythema or stinging. The aqueous gels of the present invention afford more control in application, and better maintenance of a uniform distribution of the drug over the area to be treated, than would generally be expected if the drug were applied as a cream or in an aqueous solution.

The ideal vehicle advantageously function as a "sustained delivery" system for the drug, in which the drug continously is delivery to the cells at, or slightly above, a minimum therapeutically effective level which is sustained over a period of time. This mode of drug release from the vehicle is preferred over vehicles which release the drug at levels much higher than the necessary therapeutic level shortly after application to the skin, followed by a sharp decrease to a level which is not therapeutically effective. The aqueous gels of the present invention function as sustained delivery systems, whereas prior art formulations generally do not provide sustained drug delivery at a relatively constant therapeutically effective level over a period of time.

The polymer may be any suitable polymer which is hydrophilic, has free carboxylic groups and high base binding capacity, forms a gel of uniform consistency when neutralized with a base, and is effective in promoting solubility of metronidazole in the resulting gel. Preferred polymers for use in the formulations of the invention are high molecular weight polycarboxylated vinyl polymers, with polyacrylic acid polymers being particularly preferred. The molecular weight of the polymer is desirably between 1,250,000 and 3,000,000. Suitable polyacrylic acid polymers include those commercially available from B.F. Goodrich, Cincinatti, Ohio, under the trademarks Carbopol 934, 940, and 941. Carbopol 940™ is a particularly preferred polymer for use in this invention.

The polymer is present in an amout sufficient to promote solubility of the drug in water and to cause gelling of the formulation, imparting the desired viscous consistency to the topical formulation. The metronidazole formulations advantageously comprise 0.2% to 7.0% by weight of the polymer, preferably 0.5% to 1.5%, and most preferably substantially 0.6% by weight of the polymer.

Aqueous solutions of these polymers are known to form gels when neutralized with a base. Water-soluble bases which have been used to promote gelling of polymers such as carbopols include organic amines (alkylamines such as methylamine and ethylamine, dialkylamines, trialkylamines, alkanolamines, and dialkanolamines, among others) and inorganic bases such as an aqueous solution of ammonia. The drug being added to the formulation of the present invention, metronidazole, is sufficiently basic to partially neutralize the acidic polymer in aqueous solution to the desired degree and to promote gelling.

Metronidazole is employed in the compositions in an effective amout. The actual concentration of the drug may vary, depending on the nature and degree of the disorders being treated, and whether the drug in being administered for therapeutic or prophylatic purposes. The formulations advantageously comprise from 0.1% to 1.5%, preferably 0.25% to 1.0%, and most preferably substantially 0.75% by weight of metronidazole.

Optionally, the formulation may also comprise a "penetration enhancer", i.e., an agent that promotes penetration of the active drug into the patient's skin or tissues. Such penetration ennancers include but are not limited to, dimethyl sulfoxide (DMSO) and propylene glycol with the latter being preferred. The composition advantageously comprises from 1.0% to 50%, preferably 2% to 5%, and most preferably substantially 3% by weight of the penetration enhancer.

Preservatives optionally may be incorporated into the compositions in an amout effective for inhiviting growth of microbes such as yeast and molds in the composition during storage. Any conventional preservatives may be used, with parabens being preferred. A mixture of methyl paraben and propyl paraben has been found particularly effective as a preservative. Most preferably, the composition comprises substantially 0.08% by weight of methyl paraben and substantially 0.02% by weight of propyl paraben.

Ethylenediaminetetraacetic acid (EDTA) or one of its salts is commonly added to dermatological preparations, and may optionally be incorporated into the compositions of the present invention. EDTA chelates certain metals that may be present in the formulation, which is useful because some patients have adverse reactions to preparations containing metal impurities. The EDTA will also inhibit undesirable "browning" of the composition which may occur over time in compositions having low pH (e.g., pH 3.5 to 5.4). Advantageously, the formulation of the invention comprises from 0.01% to 0.1%, preferably substantially 0.05% by weight of EDTA.

The final pH of the formulations of the invention may vary within a physiologically compatible range. Advantageously, the final pH is a physiologically compatible (i.e., not harmful to biological tissue) acidic pH. The pH is preferably between 3 and 6.9, and most preferably between 4 and 5. Any suitable method of adjusting the pH of aqueous solutions may be used. Advantageously, sodium hydroxide (NaOH) is added to the formulation to bring the final pH to the desired level. Gel formulations of the invention are more viscous at pH values that approach neutrality than at the more acidic pH values within the preferred range (i.e., viscosity increases as the polymer in the gel is neutralized to greater degrees, e.g. with NaOH).

The ingredients listed above may be combined in any order and manner that produces a formulation comprising metronidazole dissolved in (and evenly dispersed throughout) a one-phase aqueous gel of the desired consistency and pH. One suitable method of preparing formulations of the invention involves preparation of an aqueous solution of the polymer, which will be called "Part A". Advantageously, this solution comprises the polymer in distillepd water. A "Part B" is prepared comprising metronidazole. Mixing of Parts A and B results in gelling of the composition. The optional penetration enhancer and preservative(s) are preferably included in Part B. If EDTA is to be added to tie formulation, it is preferably included in Part A. The pH may then be adjusted to the desired level, e.g., by addition of NaOH.

The resulting homogeneous gels possess the advantageous properties described above, including non-comedogenic, non-acneogenic, and non-irritating ingredients ; higher specific activity of metronidazole due to increased diffusion across membranes and release from the vehicle (resulting in greater therapeutic effectiveness using smaller amounts of the drug) ; and a desirable consistency that prevents undesired pooling and spreading of the drug. High concentrations of skin-drying ingredients (e.g. alcohols and acetone), which are found in some dermatological preparations to promote drug solubility, are also avoided. Such ingredients at high concentration may excessively dry the patient's skin, causing undesirable flaking and discomfort.

The therapeutic effectiveness of the metronidazole formulations of the present invention is demonstrated in the following examples. These examples are meant to illustrate the invention rather than to limit its scope.

For example, additional ingredients such as colouring agents and sunscreens, may be included in the formulations as long as the resulting formulation retains the desirable properties (non-comedogenicity, high specific activity, etc.) described above.

The drug 1-(2-hydroxyethyl)-2-methyl-5-nitroimidazole and various derivatives thereof are described in U.S. Patent No. 2,944,061. The term "metronidazole" as used in this application is meant to include not only 1-(2-hydroxyethyl)-2-methyl-5-nitroimidazole, but also those analogues and derivatives of the drug which are solubilized in the gel formulations previously described and which have therapeutic activity when topically applied.

## Example I

A 30 kg batch of a formulation of the present invention was prepared as follows. 0.18 kg of Carbopol® 940 (0.6% by weight of the final weight of the formulation) was dissolved in 16.5 liters of distilled water containing 15 g of EDTA disodium dihydrate. Sufficient 10% NaOH was added to bring the pH to substantially 5. This aqueous polymer solution was called "Part A". "Part B" was prepared by mixing 0.9 kg of propylene glycol (3% by weight of the final weight) with 24 g of methyl paraben (0.08% by weight as above), 6.0 g of propyl paraben (0.02% by weight as above) and adding to 0.225 kg of metronidazole in 11.4 l of distilled water maintained at 50°C. Parts A and B were then mixed thoroughly and gelling of the formulation resulted. A cold aqueous solution of NaOH was then used to adjust the final pH to 5.25, and distilled water was added to give the desired 30 kg final weight. The NaOH and water were thoroughly mixed into the viscous gel.

Example II

A randomized, double blind, placebo controlled clinical trial was conducted to demonstrate the positive clinical efficacy of the aqueous metronidazole-containing gel formulation prepared in Example I in treating rosacea. The study included patients who had received no prior treatment for rosacea, as well as patients who had been treated by conventional methods. Patients discontinued treatment, if any, at least 21 days prior to the start of this study. Each patient received metronidazole in the gel formulation on one side of the face and the formulation gel (placebo control) without metronidazole on the other side of the face. Therefore, in this study, each patient served as their own control.

The effectiveness of the treatment was rated, at the time points indicated in the Tables below, in six different categories, namely, reduction in inflammatory lesions (papules and pustules), erythema, stinging, burning, itching, and dryness. The data are shown in the Tables below.

Table I-A shows the percent reduction in inflammatory lesions (papules and pustules) from baseline values for active (i.e. metronidazole-treated) and placebo-treated sides. Inflammatory lesions were progressively reduced from 46.7% to 59.9% for active-treated sides while placebo-treated sides reflected an exacerbation. There was an 82.6% difference in inflammatory lesions at the end of drug treatment on the metronidazole versus placebo-treated sides.

Table I-B shows mean erythema values for active- and placebo-treated sides. Statistically significant difference were found at visits 2, 3, 4 and 5 for the active sides and at visits 3 and 4 for the placebo sides, when compared to baseline values. Active- and placebo-side values were significantly different from each other at visits 3, 4 and 5. A concommitant improvement in reduction of erythema was seen at the same time point on the treated side and on the placebo side, both statistically significant from baseline with the metronidazole treated side significantly more improved than the placebo side.

Table II-A shows mean stinging scores for active-and placebo-treated sides. Statistically significant differences were found at visits 3, 4 and 5 for both the active and placebo sides, when compared to baseline values. Active- and placebo-side values were not significantly different from one another.

Table II-B shows mean burning scores for active- and placebo-treated sides. Statistically significant differences were found at all visits (2, 3, 4, 5) for both the active and placebo sides, when compared to baseline values. Active- and placebo-side values were not significantly different from one another.

Table II-C shows mean itching scores for active- and placebo-treated sides. Statistically significant differences were found at all visits (2, 3, 4, 5) for both the active and placebo sides, when compared to baseline values. Active- and placebo-side values were not significantly different from one another.

Table II-D shows means dryness scores for active- and placebo-treated sides. Statistically significant differences were found at visits 3, 4 and 5 for active sides and at visits 4 and 5 for placebo sides, when compared to baseline values. Active- and placebo-side values were not significantly different from one another.

Tables II-A, II-B, II-C and II-D show an unexpected out dramatic improvement in local tolerance data. This data represents the patients' subjective assessments of stinging, burning, itching and dryness on each side of their faces before and during drug or placebo treatment. The data shows that there was a dramatic (highly statistically significant) improvement in the patients' perceptions of these attendant complications of the disease. Since both sides improved to the same degree (no statistically significant difference can be found), the improvement apparently comes from the gel formulation per se.

This data confirms the effectiveness of metronidazole in the gel formulation for treatment of acne rosacea and also demonstrates the unique therapeutic effects of the gel formulation.

## Table I-A

### Inflammatory Lesions
### (Efficacy Data From 20 Subjects)

| | On Drug | | | Off Drug |
|---|---|---|---|---|
| | Visit 2 Weeks 3-5 | Visit 3 Weeks 6-8 | Visit 4 Weeks 9-11 | Visit 5 Weeks 12-17 |
| Active (Percent Reduction from Baseline) | 46.7 | 55.1 | 59.9 | 41.6 |
| Placebo (Percent Reduction from Baseline) | -22.5 | - 4.2 | -22.7 | -46.8 |
| Difference (Active-Placebo Percent Difference) | '69.2 | 59.3 | 82.6 | 88.4 |

## Table I-B

### Erythema
### (Efficacy Data From 20 Subjects)

| | Visit 1 Baseline | On Drug | | | Off Drug |
| | | Visit 2 Weeks 3-5 | Visit 3 Weeks 6-8 | Visit 4 Weeks 9-11 | Visit 5 Weeks 12-17 |
|---|---|---|---|---|---|
| Active (Mean Values) | 2.10 | 1.55*** | 1.05*** | 1.05*** | 1.15*** |
| Placebo (Mean Values) | 2.10 | 1.90 | 1.55*** | 1.55*** | 1.70 |

3=Severe
2=Moderate
1=Mild
0=Absent

\*** p<.01 compared to baseline values.

| Active Versus Placebo Significant Differences (p values) | No Difference | No Difference | p<.02 | p<.02 | p<.02 |

## Table II-A

### Stinging
### (Local Tolerance Data from 20 Subjects)

| | Visit 1 Baseline | On Drug | | | Off Drug |
| | | Visit 2 Weeks 3-5 | Visit 3 Weeks 6-8 | Visit 4 Weeks 9-11 | Visit 5 Weeks 12-17 |
|---|---|---|---|---|---|
| Active (Mean Values) | 0.70 | 0.25 | 0.15** | 0.00*** | 0.00*** |
| Placebo (Mean Values) | 0.65 | 0.30 | 0.15* | 0.20* | 0.05*** |

3=Severe
2=Moderate
1=Mild
0=Absent

  * p<.05 compared to baseline values
 ** p<.02 compared to baseline values
*** p<.01 compared to baseline values

| Active Versus Placebo Significant Differences (p values) | No Difference | No Difference | No Difference | No Diffrence | No Difference |
|---|---|---|---|---|---|

## Table II-B

### Burning

(Local Tolerance Data from 20 Subjects)

| | Visit 1 Baseline | On Drug | | | Off Drug |
|---|---|---|---|---|---|
| | | Visit 2 Weeks 3-5 | Visit 3 Weeks 6-8 | Visit 4 Weeks 9-11 | Visit 5 Weeks 12-17 |
| Active (Mean Values) | 1.25 | 0.30** | 0.10*** | 0.05*** | 0.05*** |
| Placebo (Mean Values) | 1.05 | 0.30*** | 0.05*** | 0.10*** | 0.05*** |

3=Severe
2=Moderate
1=Mild
0=Absent

** p<.02 compared to baseline values
*** p<.01 compared to baseline values

| Active Versus Placebo Significant Differences (p values) | No Difference | No Difference | No Difference | No Diffrence | No Difference |

## Table II-C

### Itching
#### (Local Tolerance Data from 20 Subjects)

|  | Visit 1 Baseline | On Drug | | | Off Drug |
|---|---|---|---|---|---|
|  |  | Visit 2 Weeks 3-5 | Visit 3 Weeks 6-8 | Visit 4 Weeks 9-11 | Visit 5 Weeks 12-17 |
| Active (Mean Values) | 1.45 | 0.55*** | 0.15*** | 0.20*** | 0.10*** |
| Placebo (Mean Values) | 1.40 | 0.70*** | 0.25*** | 0.20*** | 0.15*** |

3=Severe
2=Moderate
1=Mild
0=Absent

\*\*\* p<.01 compared to baseline values

| Active Versus Placebo Significant Differences (p values) | No Difference | No Difference | No Difference | No Diffrence | No Difference |
|---|---|---|---|---|---|

## Table II-D

### Dryness
#### (Local Tolerance Data from 20 Subjects)

|  | Visit 1 | On Drug | | | Off Drug |
|---|---|---|---|---|---|
|  |  | Visit 2 | Visit 3 | Visit 4 | Visit 5 |
|  |  | Weeks | Weeks | Weeks | Weeks |
|  | Baseline | 3-5 | 6-8 | 9-11 | 12-17 |
| **Active** | | | | | |
| (Mean Values) | 1.45 | 0.85 | 0.50*** | 0.25*** | 0.25*** |
| **Placebo** | | | | | |
| (Mean Values) | 1.40 | 0.85 | 0.75 | 0.20*** | 0.45*** |

3=Severe
2=Moderate
1=Mild
0=Absent

*** $p < .01$ compared to

baseline values

| **Active Versus Placebo Significant Differences (p values)** | No Difference | No Difference | No Difference | No Diffrence | No Difference |
|---|---|---|---|---|---|

## Claims

1. A dermatological preparation for topical application in the form of an aqueous gel composition consisting essentially of
a) a therapeutically effective amout of metronidazole,
b) a polycarboxylated vinyl polymer in an amout effective to promote solubility of the metronidazole and to cause gelling of the composition, and
c) an aqueous solvent, and
d) from 2% to 5% by weight of a penetration enhancer, wherein the preparation is substantially free of comedogenic, acneogenic, irritating and skin-drying ingredients.

2. The composition of claim 1 wherein the concentration of metronidazole is from 0.1% to 1.5% by weight.

3. The composition of claim 2 wherein the concentration of metronidazole is from 0.25% to 1.0% by weight.

4. The composition of claim 3 wherein the concentration of metronidazole is substantially 0.75% by weight.

5. The composition of claim 1 wherein the polymer is present in an amout of from 0.2% to 7.0% by weight.

6. The composition of claim 5 wherein the polymer is present in an amout of from 0.5% to 1.5% by weight.

7. The composition of claim 6 wherein the polymer is present in an amout of substantially 0.6% by weight.

8. The composition of claim 1 wherein the polymer is a hydrophilic, high-molecular weight polycarboxylated vinyl polymer.

9. The composition of claim 8 wherein the polymer is a polyacrylic acid polymer.

10. The compositon of claim 9 wherein the molecular weight of the polymer is from 1,250,000 to 3,000,000 daltons.

11. The composition of claim 1 which additionally includes an amout of ethylenediaminetetraacetic acid (EDTA) sufficient to chelate metal impurities present.

12. The composition of claim 1 wherein the concentration of the penetration enhancer is substantially 3%

EP 0 305 380 B1

by weight.

13. The composition of claim 1 wherein the penetration enhancer is DMSO or propylene glycol.

14. The composition of claim 1 which additionally includes an effective amout of a preservative.

15. The composition of claim 14 wherein the preservative consists of one or more parabens.

16. The composition of claim 15 wherein the preservative consists of substantially 0.08% by weight of methyl paraben and substantially 0.02% by weight of propyl paraben.

17. The composition of claim 1 wherein the pH of the composition is a physiologically compatible acidic pH.

18. The composition of claim 17 wherein the pH is between 3 and 6.9.

19. The composition of claim 18 wherein the pH is between 4 and 5.

20. The composition of claim 1 wherein the aqueous solvent is distilled water.

## Patentansprüche

1. Dermatologisches Mittel zur topischen Applikation in Form einer wäßrigen Gelzusammensetzung, die im wesentlichen besteht aus

a) einer therapeutisch wirksamen Menge Metronidazol,

b) einem polycarboxylierten Vinylpolymeren in einer Menge, die wirksam ist, die Löslichkeit des Metronidazols zu fördern und das Gelieren der Zusammensetzung zu bewirken,

c) einem wäßrigen Lösungsmittel und

d) 2 bis 5 Gew.-% eines Penetrationsverstärkers, worin das Mittel im wesentlichen frei von comedogenen, akneigenen, reizenden und die Haut austrocknenden Reagenzien ist.

2. Zusammensetzung nach Anspruch 1, worin die Metronidazolkonzentration 0,1 bis 1,5 Gew.-% beträgt.

3. Zusammensetzung nach Anspruch 2, worin die Metronidazolkonzentration 0,25 bis 1,0 Gew.-% beträgt.

4. Zusammensetzung nach Anspruch 3, worin die Metronidazolkonzentration etwa 0,75 Gew.-% beträgt.

5. Zusammensetzung nach Anspruch 1, worin das Polymer in einer Menge von 0,2 bis 7,0 Gew.-% vorliegt.

6. Zusammensetzung nach Anspruch 5, worin das Polymer in einer Menge von 0,5 bis 1,5 Gew.-% vorliegt.

7. Zusammensetzung nach Anspruch 6, worin das Polymer in einer Menge von etwa 0,6 Gew.-% vorliegt.

8. Zusammensetzung nach Anspruch 1, worin das Polymer ein hydrophiles polycarboxyliertes Vinylpolymer mit hohem Molekulargewicht ist.

9. Zusammensetzung nach Anspruch 8, worin das Polymer ein Polyacrylsäurepolymer ist.

10. Zusammensetzung nach Anspruch 9, worin das Molekulargewicht des Polymers 1250000 bis 3000000 Dalton beträgt.

11. Zusammensetzung nach Anspruch 1, die zusätzlich eine Menge Ethylendiamintetraessigsäure (EDTA) enthält, die ausreichend ist, um vorliegende Metallverunreinigungen zu chelatisieren.

12. Zusammensetzung nach Anspruch 1, worin die Konzentration des Penetrationsverstärkers etwa 3 Gew.-% beträgt.

13. Zusammensetzung nach Anspruch 1, worin der Penetrationsverstärker DMSO oder Propylenglykol ist

14. Zusammensetzung nach Anspruch 1, die zusätzlich eine wirksame Menge eines Konservierungsmittels enthält.

15. Zusammensetzung nach Anspruch 14, worin das Konservierungsmittel aus einem oder mehreren Paraben(en) besteht.

16. Zusammensetzung nach Anspruch 15, worin das Konservierungsmittel etwa aus 0,08 Gew.-% Methylparaben und etwa aus 0,02 Gew.-% Propylparaben besteht.

17. Zusammensetzung nach Anspruch 1, worin der pH-Wert der Zusammensetzung ein physiologisch verträglicher saurer pH-Wert ist.

18. Zusammensetzung nach Anspruch 17, worin der pH-Wert zwischen 3 und 6,9 liegt.

19. Zusammensetzung nach Anspruch 18, worin der pH-Wert zwischen 4 und 5 liegt

20. Zusammensetzung nach Anspruch 1, worin das wäßrige Lösungsmittel destilliertes Wasser ist.

## Revendications

1. Préparation dermatologique pour application topique sous la forme d'une composition de gel aqueux constituée pour l'essentiel par :

a) une quantité thérapeutiquement efficace de métronidazole,

b) un polymère de vinyle polycarboxylé en une quantité efficace pour stimuler la solubilité du métronidazole

12

et pour causer la gélification de la composition,

c) un solvant aqueux, et

d) de 2% à 5% en poids d'un agent augmentant la pénétration, composition dans laquelle la préparation est sensiblement exemple d'ingrédients comédongènes, acnégènes, irritants et desséchant la peau.

2. Composition selon la revendication 1, dans laquelle la concentration du métronidazole est de 0,1% à 1,5% en poids.

3. Composition selon la revendication 2, dans laquelle la concentration du métronidazole est de 0,25% à 1,0% en poids.

4. Composition selon la revendication 3, dans laquelle la concentration du métronidazole est sensiblement égale à 0,75% en poids.

5. Composition selon la revendication 1, dans laquelle le polymère est présent en une quantité de 0,2% à 7,0% en poids.

6. Composition selon la revendication 5, dans laquelle le polymère est présent en une quantité de 0,5% à 1,5% en poids.

7. Composition selon la revendication 6, dans laquelle le polymère est présent en une quantité sensiblement égale à 0,6% en poids.

8. Composition selon la revendication 1, dans laquelle le polymère est un polymère de vinyle polycarboxylé, hydrophile et à poids moléculaire élevé.

9. Composition selon la revendication 8, dans laquelle le polymère est un polymère d'acide polyacrylique.

10. Composition selon la revendication 9, dans laquelle le poids moléculaire du polymère et de 1.250.000 à 3.000.000 de daltons.

11. Composition selon la revendication 1, laquelle comprend en outre une quantité d'acide éthylène-diamine tétracétique suffisants pour chélater les impuretés métalliques présentes.

12. Composition selon la revendication 1, dans laquelle la concentration de l'agent augmentant la pénétration est sensiblement égale à 3% en poids.

13. Composition selon la revendication 1, dans laquelle l'agent augmentant la pénétration est le diméthylsulfoxyde (DMSO) ou le propylène glycol.

14. Composition selon la revendication 1, laquelle comprend en outre une quantité efficace d'un agent de conservation.

15. Composition selon la revendication 14, dans laquelle l'agent de conservation est constitué par un ou plusieurs para-hydroxybenzoates.

16. Composition selon la revendication 15, dans laquelle l'agent de conservation est constitué par sensiblement 0,08% en poids de méthyl-para-hydroxybenzoate et sensiblement 0,02% en poids de propyl-para-hydroxybenzoate.

17. Composition selon la revendication 1, dans laquelle le pH de la composition est un pH acide physiologiquement compatible.

18. Composition selon la revendication 17, dans laquelle le pH est compris entre 3 et 6,9.

19. Composition selon la revendication 18, dans laquelle le pH est compris entre 4 et 5.

20. Composition selon la revendication 1, dans laquelle le solvant aqueux est de l'eau distillée.